# NEW EUROPEAN PATENT SPECIFICATION

(11) **EP 0 822 943 B2**
(45) Date of publication and mention of the opposition decision: **26.07.2006**
(45) Mention of the grant of the patent: 14.07.1999
(21) Application number: 96911123.6
(22) Date of filing: 29.04.1996
(51) Int. Cl.: C08B 37/00

(54) **INULIN DERIVATIVES**
DERIVATE VON INULIN
DERIVES D'INULINE

(30) Priority: 27.04.1995 EP 95201091
(43) Date of publication of application: 11.02.1998
(73) Proprietor: Coöperatie Cosun U.A., 4814 ND Breda (NL)
(72) Inventor: VAN BRUSSEL-VERRAEST, Dorine, Lisa, NL-2771 KS Boskoop (NL); PETERS, Johannes, Andreas, NL-2272 XE Voorburg (NL); VAN BEKKUM, Herman, NL-3136 AP Vlaardingen (NL)
(74) Representative: van Westenbrugge, Andries
(86) International application number: PCT/NL1996/000187
(87) International publication number: WO 1996/034017

(56) References cited:
- EP-A- 0 638 589
- WO-A-96/701949
- AT-A- 384 811
- FR-A- 2 178 789
- FR-A- 2 707 649
- US-A- 3 453 258
- US-A- 3 652 542
- Basics principles of organic chemistry J.D. Roberts and M.J. Caserio, Ed. W.A. Benjamin Inc. 1977

## Description

The present invention relates to new polysaccharide derivatives that can bind metals and metal salts, or have surface-active or emulsifying properties.

Polysaccharide derivatives containing side-chain functional groups are usually based on cellulose and starch, and are obtained by etherification of part or all of the free hydroxyl groups of the polysaccharide. Known derivatives include carboxymethyl cellulose, carboxyethyl cellulose and hydroxyethyl cellulose and similar starch derivatives. Although these derivatives have useful properties as binders, as complexing agents for detergents, and for paper industry and oil recovery, their relatively high viscosity and restricted solubility reduces their applicability for some purposes.

It was found that fructan derivatives, such as inulin derivatives, that are substituted with three-carbon side chains having functional groups on the side chain, can be conveniently prepared from fructan, and have useful properties as surfactants, emulsifiers and as agents inhibiting the crystal growth of calcium salts, and have low viscosity.

The invention relates to fructan derivatives which contain three-carbon side-chain groups on at least part of the free hydroxyl groups. In particular the derivatives contain 0.1 to 2.5, preferably 0.2 to 2.0 of such side-chain groups for each monosaccharide unit of the fructan. The side-chain groups have the formula -CHR¹-CHR²-R³, wherein R¹ and R² each represent hydrogen or methyl and R³ represents -CN, -C(=NX)-NR⁴R⁵, -CO-NR⁴R⁵, -CO-OR⁴, -CH₂₋NR⁵R⁶ or -CH₂-OR⁶, wherein R⁴ represents hydrogen or C₁-C₂₂-alkyl or C₁-C₂₂-alkenyl optionally substituted by hydroxy, R⁵ represents hydrogen, C₁-C₂₂-alkyl, carboxy- or hydroxy-substituted C₁-C₂₂-alkyl or optionally substituted C₁₋C₂₂-acyl, R⁶ represents hydrogen or C₁-C₂₂-acyl, and X represents hydrogen, hydroxyl or amino. In this context "alkenyl" also comprises alkadienyl, alkynyl and the like, and "acyl" comprises alkanoyl, alkenoyl, alkadienoyl, alkynoyl and the like. Different side-chains -CHR¹-CHR²-R³ may be present in a single derivative molecule, e.g. carboxyethyl groups and carbamoylethyl groups.

The term fructan is used here to denote any polysaccharide of at least three monosaccharide units, wherein the majority of the monosaccharide units consists of anhydrofructose units. The most prominent example of fructans is inulin. Inulin is a polydisperse mixture of polysaccharides, consisting of 2,1-β-linked anhydrofructose units having a glucose unit at the reducing end. It is often branched trough 2,6-β linkages. It can be obtained e.g. from Jerusalem artichoke, artichoke, chicory, dahlia and dandelion. Depending on its origin, it has a chain length of 5-30 or higher.

The fructans that are covered by the present invention further include levan (a 2,6-fructan) or short-chain analogues thereof, and synthetic fructose oligosaccharides. The fructans to be used as a staring material for the derivatives of the invention also comprise physically or chemically pretreated or derivatised inulin and levan, such as partially hydrolysed or partially oxidised inulin.

Preferred fructan derivatives are those wherein R¹ and R² each represent hydrogen. Further preferred are derivatives wherein R³ represents -CN, -C(=NOH)-NH₂, -CO-NR⁴R⁵, -CO-OR⁴ or -CH₂-NR⁵R⁶, wherein R⁴ represents hydrogen or C₁₂-C₂₀-alkyl or C₁₂-c₂₀-alkenyl optionally substituted by hydroxy, R⁵ represents hydrogen, hydroxymethyl, hydroxyethyl, carboxymethyl or substituted aminoacetyl or R⁵ or R⁶ represents hydrogen or C₁₂-C₂₀-acyl. Examples of substituted aminoacetyl as R⁵ (to be used e.g. when R³ is -CH₂-NR⁵R⁶) are N,N-bis(carboxymethyl)aminoacetyl, N,N',N'-tris(carboxymethyl)aminoethyl-aminoacetyl and 3,6,9-tris(carboxymethyl)-3,6,9-triazaundecanedioyl. Particularly preferred are derivatives wherein R³ represents -CONH₂ or -COOH. It should be understood that compounds having carboxyl groups -COOH also comprise the metal and ammonium salts of such compounds, whereas compounds having amino groups also comprise the acid addition salts of such compounds.

The fructan derivatives according to the invention can be prepared e.g. by base-catalysed addition of an acrylic derivative to the fructan. Examples of acrylic derivatives are acrylic, methacrylic, crotonic, nitriles, amides and esters. In particular the derivatives can be obtained by addition of a nitrile having formula R¹HC=CR²-CN, an amide having formula R¹HC=CR²-CO-NR⁴R⁵, or an ester having formula R¹HC=CR²-COOR⁷, wherein R¹, R², R⁴ and R⁵ are as defined above and R⁷ represents hydrogen or C₁-C₄-alkyl.

In case of an addition of acrylonitrile, the hydroxyl groups of the polysaccharide react with acrylonitrile in the presence of alkali to form a cyanoethyl ether. The reaction, which is a nucleophilic addition of the Michael-type, may be presented as follows:

Polysaccharide-OH + CH₂=CH-CN → Polysaccharide-O-CH₂-CH₂-CN

The addition reaction can also be performed e.g. with acrylamide or an N-substituted acrylamide, or with acrylic acid or a lower alkyl ester thereof. The addition of acrylonitrile is preferred because it gives the highest yield. Moreover, the nitrile groups are reactive functional groups which can be converted into other functional groups, such as amines via reduction, or amides and carboxylates via hydration.

Some of the fructan derivatives according to the invention can also be prepared by addition of cyclic three-carbon units to the fructan. This applies e.g. to the preparation of the carboxyethyl derivative by addition of β-propionolactone to the fructan.

As an illustration of the process according to the invention, the cyanoethylation reaction is discussed in some more detail below:

The fructan is dissolved in water, preferably in a minimum amount of water in order to avoid side reactions of acrylonitrile with water in alkaline medium, which may be presented as follows:

CH₂=CH-CN + H₂O → HO-CH₂-CH₂-CN

CH₂=CH-CN + HO-CH₂-CH₂-CN → O-(CH₂-CH₂-CN)₂

The extent to which these side reactions occur determines the efficiency of the cyanoethylation. The acrylonitrile (AN) efficiency (A.E.) can be expressed as the percentage of acrylonitrile consumed for cyanoethyl-fructan formation and can be determined by the analysis of the degree of substitution (ds) of the product using ¹H NMR spectroscopy. In order to optimise the reaction conditions for the highest A.E., the influences of reaction temperature, reaction time, hydroxide concentration and molar ratio of AN:fructan were studied.

The reaction can be carried out at varying temperatures, preferably within the range of 20-60°C, more preferably within the range of 35-50°C. At such reaction temperatures the reaction times are relatively short, in general from several (e.g. 5) minutes to 1 hour. Longer reaction times do not normally result in higher yields. The preferred alkali concentration is 0.5-20 mmol/l, especially 1.0-10 mmol/l. At low NaOH concentration (< 0.3 mol/l), the A.E. was low. In all cases, cyanoethyl ethers were formed with a high selectivity for cyano groups: no hydration products (amide or carboxylate), which may be formed under alkaline conditions, could be detected (¹³C NMR spectroscopy). The optimum results (A.E. up to 90 %) were obtained with a molar ratio [Inulin (monosaccharide units) : water: NaOH] = [1 : 15-25 (17) : 0.1-0.3 (0.2)], a temperature of 45°C and a reaction time of 30 min.

The ds of the produced cyanoethyl fructan can be tuned by varying the molar ratio of AN:fructan. The solubility of the products in various solvents depends strongly on the ds. Products with a low ds (ds = 0.25-1.00) are more water soluble (> 0.5 g/ml) than the starting material fructan. Products with a higher ds (ds = 1.00-1.50) are insoluble in cold water and soluble in hot water and in methanol-water mixtures. When the ds is > 1.5, the products are insoluble in water and soluble in methanol-water mixtures, methanol and acetone.

The cyanoethyl-fructan can be converted to a variety of other derivatives according to the invention. Where reference is made below to cyanoethyl, carboxyethyl and the like, the same applies *mutatis mutandis* to the methyl homologues thereof such as the 2-cyanopropyl, 2-carboxypropyl, 1-methyl-2-cyanoethyl and 1-methyl-2-carboxyethyl derivatives.

First, the nitrile group can be reduced to a primary amine using a suitable reducing agent. A suitable reducing agent is sodium borohydride in the presence of a transition metal salt, e.g. cobaltous chloride hexahydrate, preferably at ambient temperatures (10-30°C). The aminopropyl-fructan can also be prepared by catalytic hydrogenation of the cyanoethyl-fructan using transition metal catalysts, such as Raney Ni, Ru/C or preferably Raney Co, or by reduction of cyanoethyl-fructan with a metal (e.g. sodium, lithium, calcium) in liquid ammonia and methanol.

The cyanoethyl-fructan can also be converted to an amidoxime (hydroxyamidino-ethyl derivatives) with the formula -O-CHR¹-CHR²-C(=NOH)-NH₂ by reaction with hydroxylamine. Similarly, the corresponding amidines (-C(=NH)-NH₂) and amidrazones (-C(=NNH₂)-NH₂) may be obtained.

Furthermore, the cyanoethyl-fructan can be converted to carbamoyl-ethyl and carboxyethyl derivatives by hydrolysis of the nitrile group. The hydrolysis is performed under relatively mild conditions in order to avoid glycosidic bond cleavage in acidic medium or decyanoethylation in strong alkaline medium. Mild conditions can be applied when hydrogen peroxide is used in alkaline medium. The peroxide anion is a strong nucleophile and accelerates the hydration. Cyanoethyl-fructan is converted to the polyamide when the hydration is carried out e.g. with 2 equivalents of H₂O₂ at pH 9.5 and 60°C for 1 hour. The extent of decyanoethylation under these conditions is minor (5 to 6 %).

The carbamoylethyl compounds may be hydrolysed to the carboxyethyl compounds using alkaline (pH 11-14) conditions and at elevated temperatures, e.g. 60-100°C; the reaction may be enhanced by addition of sodium hydroperoxide or sodium peroxide.

The carboxyethyl, carbamoylethyl, aminopropyl, hydroxypropyl derivatives can be further converted to their esters and amides, by reaction with the appropriate alkyl alcohol, alkyl amine, alkyl halide, acyl halide or the like, in a way known per se. For example, the carboxyethyl derivative can be converted to the octadecyl ester by esterification using octadecanol, optionally via the acid chloride. As another example, a 2-hydroxyhexadecyl ester can be obtained by reaction of the carboxyl derivative with 1-epoxyhexadecane. Also, the carbamoylethyl derivative can be N-hydroxymethylated with formaldehyde.

The aminopropyl compound can be converted to the corresponding stearoyl amide using stearoyl chloride. The aminopropyl compound can also be converted to the carboxymethylaminopropyl derivative by reaction with sodium chloroacetate, or to the 3,6,9,9-tetrakis(carboxymethyl)-3,6,9-triazanonanoyl-aminopropyl and/or the 3,6,9-tris(carboxymethyl)-3,6,9-triazaundecanedioyl-bis-aminopropyl derivative by reaction with diethylenetriaminepentaacetic (DTPA) bisanhydride.

The cyanoethylated products are useful as a starting material for other C3-extended fructan derivatives, and as an ingredient in textile treatment; they also possess good dispersing properties. The hydroxyamidino-ethylated products have strong chelating properties and may be used for the extraction or removal of metal ions.

The carboxy-ethylated, carbamoylethylated and mixed carboxyethylated/carbamoylethylated products may be applied in a variety of fields, e.g. as sequestering agent in detergent formulations, and are excellent crystallisation inhibitors inhibiting the precipitation of sparingly soluble salts, such as calcium carbonate. They can also be used as a metal ion carrier or as a dispersing agent. Products having mixed carbamoylethyl and carboxyethyl groups are also suitable as hair fixatives.

The N-carboxymethyl-aminopropylated products have similar applications. The EDTA- and DTPA-derived products, especially the 3,6,9-tris(carboxymethyl)-3,6,9-triazaundecanedioyl-bis-aminopropyl derivative having inulin residues at both ends, is an excellent contrast agent for diagnostic purposes such as a magnetic resonance imaging agent, when complexed with a lanthanide metal such as Gd or Dy.

The esters and amides having long alkyl (alkenyl) chains are very useful as surfactants and emulsifiers.

### EXAMPLES

**Materials**. Two types of inulin were used. The first type (Type l), with an average dp = 30, was obtained from E. Merck (Darmstadt). The second type (Type II), isolated from chicory root, is commercially available under the name "Frutafit®" and has an average dp of about 10.

### Analytical Methods.

NMR spectroscopy: ¹³C NMR and ¹H spectra were recorded on a Nicolet NT-200 WB and a Varian VXR-400 S spectrometer using D₂O as solvent and t-butyl alcohol as internal standard. For the non-water-soluble products DMSO-*d*_{*6*} was used as solvent.

### Example 1: Cyanoethylation of inulin:

Water-soluble cyanoethyl-inulin: Inulin (3.3 g, 20 mmol monomeric units) was dissolved at elevated temperature in a minimum amount of water (about 5 ml) in a 100 ml stoppered flask. Then NaOH (4 mmol) and acrylonitrile were added, the flask was stoppered and heated at 45°C during 30 min. The reaction was stopped by neutralising the reaction mixture with 2 M HCl. The remaining acrylonitrile and byproducts such as cyanohydrin were evaporated under reduced pressure. Low molecular weight material (MW < 200), such as NaCl, was removed by membrane filtration (UTC 60, Toray Industries Inc., Tokyo) at a pressure of 20 bar. Finally, the product was freeze-dried.

Water-insoluble cyanoethyl-inulin: Cyanoethyl inulin with a high degree of substitution (ds > 1.5) is insoluble in water. The procedure for the preparation is similar to the preparation of water-soluble cyanoethyl-inulin. At the end of the reaction, the product precipitates as a white solid. After neutralisation of the mixture, the precipitate was collected by filtration, washed and dried.
With these reaction conditions, cyanoethyl inulin was prepared with high efficiency. The efficiency is defined as the ratio of the amount of acrylonitrile consumed during the cyanoethyl-inulin formation to the amount of acrylonitrile added to the reaction mixture. When 20 mmol inulin (monomeric units) was reacted with 10 mmol acrylonitrile, cyanoethyl inulin with a ds=0.45 (efficiency = 90%) was obtained (yield 85 %).

Structure analysis and determination of degree of substitution: The structure of the reaction product was confirmed using ¹³C NMR spectroscopy (δ (ppm) 19.7 - 20.3 (CH₂CN), 61.0 - 85.0 (C1, C3-C6 and OCH₂), 104.5 - 105.2 (C2), 121.3 -121.5 (CN). The obtained degree of substitution (ds) was determined via ¹H NMR spectroscopy (δ (ppm) 2.7 - 2.9 (CH₂CN), 3.3 - 4.5 (H1a-b, H3-H5, H6a-b and OCH₂), 5.3 - 5.5 (H1 of glucose unit).

### Example 2: Reduction of cyanoethyl-Inulin:

The nitrile groups of water soluble cyanoethyl inulin were reduced into primary amines using sodium borohydride in the presence of cobaltous chloride hexahydrate, i.e. cyanoethyl inulin (1 g) and cobaltous chloride hexahydrate (4 equivalents with respect to cyanoethyl groups) were dissolved in 20 ml water and sodium borohydride (15 equivalents with respect to cyanoethyl groups) was added in portions with stirring at 20°C. Evolution of hydrogen gas was observed and a black precipitate (due to metallic Co) appeared during the reaction. When the addition was complete, stirring was continued for one hour. The black precipitate was filtered off and washed with water. Borate, which was formed from sodium borohydride, was removed using membrane filtration (UTC 60, Toray Industries) at a pressure of 20 bar. The conversion of nitrile groups into primary amine groups was 90 %. The structure of the product was established using ¹³C NMR spectroscopy: (δ (ppm) 29.5 - 32.0 (CH₂CH₂NH₂), 38.0 - 40.0 (CH₂CH₂NH₂), 61.0 - 85.0 (C1, C3-C6 and OCH₂), 104.5 - 105.2 (C2), and by ¹H NMR spectroscopy with a H₂O/D₂O mixture as solvent (δ(ppm) 6.8 - 7.0 and 7.4 - 7.6 (CH₂NH₂)). The degree of substitution (ds) was determined via ¹H NMR spectroscopy (δ (ppm) 1.7 - 2.0 (CH₂CH₂NH₂), 2.9 - 3.1 (CH₂CH₂NH₂), 3.3 - 4.5 (H1a-b, H3-H5, H6a-b and OCH₂), 5.3 - 5.5 (H1 of glucose unit).

### Example 3: Preparation of carbamoylethyl-inulin from cyanoethyl-inulin:

The nitrile groups of water-soluble cyanoethyl-inulin were selectively hydrated to amide groups as follows: Cyanoethyl-inulin (5 g) was dissolved in 10 ml water. A 35 % H₂O₂ solution (2 mol equivalent with respect to cyanoethyl groups) was added. The hydration was carried out at 60°C and pH 9.5. The pH was kept constant by adding 0.1 M aqueous NaOH from an automatic burette (Metrohm 655 Dosimat), which was used in combination with a Metrohm 614 Impulsomat coupled to a Metrohm 605 pH meter. After about 1 h, the reaction was complete. The reaction mixture was neutralised and the product was purified using membrane filtration (UTC 60, Toray Industries) at 20 bar. The structure of the reaction product was established using ¹³C NMR spectroscopy (δ (ppm) 36.0-37.5 (CH₂CO), 61.0-87.0 (C1, C3-C6 and OCH₂), 104.5-105.2 (C2), 178.3-178.6 (CONH₂) and by ¹H NMR (H20/D20 9:1) δ (ppm) 6.8-7.0 and 7.4-7.6 (CONH₂). The ds was determined by ¹H NMR (δ (ppm) 2.3 - 2.6 (CH₂CONH₂), 3.3-4.5 (H1a-b, H3-H5, H6a-b and OCH₂), 5.3-5.5 (H1 of glucose unit).

### Example 4: Preparation of carbamoylethyl-inulin from inulin:

Carbamoylethyl-inulin was prepared in one step by etherification with acrylamide. Inulin (3.3 g, 20 mmol monomeric units) was dissolved in 5 ml water. Then NaOH (4 mmol) and subsequently acrylamide (2.84 g, 40 mmol) were added. The reaction mixture was heated at 50°C for 2 h. The reaction was stopped by neutralizing with 2 M HCl. The remaining acrylamide and low molecular weight material (MW < 200) were removed by membrane filtration. The product was freeze-dried. Carbamoylethyl-inulin (3.5 g) with a ds of 0.97 was obtained.

### Example 5: Preparation of carboxyethyl-inulin:

The amide groups of carbamoylethyl-inulin were hydrolysed to carboxylate groups as follows: Carbamoylethyl inulin with ds=0.95 (0.5 g, 2.18 mmol amide groups) was dissolved in 10 ml water and heated at 65°C. Sodium peroxide (340 mg, 4.36 mmol) was added in small portions. After 2.5 h, the reaction mixture was neutralized and the product was purified using membrane filtration. Thus, carboxyethyl-inulin (0.33 g) with a ds of 0.50 was obtained. The structure of the reaction product was established using ¹³C NMR spectroscopy (δ (ppm) 39.0 - 39.8 (CH2COO), 61.0 - 87.0 (C1, C3-C6 and OCH₂), 104.5 - 105.2 (C2), 181.5 - 183.0 (COO). The ds was determined using ¹H NMR spectroscopy (δ (ppm) 2.4 -2.5 (CH₂COO), 3.3 - 4.5 (H1 a-b, H3-H5, H6a-b and OCH₂), 5.3 - 5.5 (H1 of glucose unit).

### Example 6: Preparation of carbamoylethyl-carboxyethyl inulin:

A mixed ether containing carbamoylethyl and carboxyethyl groups was obtained by partial hydrolysis of carbamoylethyl-inulin, using similar reaction conditions as in example 5, but using smaller amounts of sodium peroxide. When 1 equivalent of sodium peroxide (with respect to amide groups) was reacted with carbamoylethyl-inulin with ds = 0.94, carbamoylethyl-carboxyethyl-inulin was obtained with ds = 0.25 and 0.29 for CH₂CH₂CONH₂ and CH₂CH₂COOH groups, respectively.

### Example 7: Determination of crystallisation inhibition properties for CaCO₃

Calcium carbonate crystals were precipitated by mixing solutions containing stoichiometric amounts of calcium and carbonate ions in concentrations that are sufficient to provoke spontaneous precipitation of calcium carbonate (5*10⁻³ M at pH 10). The solutions were prepared with 0.01 M NaCl dissolved in bidistilled water and were filtered through a Millipore 0.22 µ filter before use. After mixing, the total Ca²⁺ and CO₃²⁻ concentrations were 2.5*10⁻³ M. The supersaturation ratio under these conditions was calculated to be S = 6.25. The precipitation of calcium carbonate was monitored by measuring the concentration of the free Ca²⁺ ions by a calcium selective electrode (Unicam) coupled to a Metrohm 605 pH meter. The response of the calcium selective electrode was followed during a blank precipitation (no inhibitor added) and during the precipitation of calcium carbonate in the presence of carbamoylethyl, carboxyethyl or carboxyethyl-carbamoylethyl inulin. At the beginning of the precipitation process, progressive nucleation occurs and the crystal growth is minor. Then, the decrease in concentration of free Ca-ions is not measurable. The period in which no change in concentration of free Ca-ions is measured is called induction time (t_{ind}). After that, the crystal surface available for growth is sufficiently high to cause a measurable decrease in concentration of free Ca-ions. This decrease continues until equilibrium is reached or until the growth rate becomes too low to notice further changes in the concentration of free Ca-ions, as often happens in the presence of effective growth inhibitors. The concentration of free Ca-ions at the end of the experiment, Cₑ, is then higher than the equilibrium concentration which is reached in the case no inhibitor was added. The induction period, t_{ind}, and apparent equilibrium concentration of free Ca-ions, Cₑ, observed for the crystallisation of calcium carbonate from a solution with pH=10 and a supersaturation ratio S = 6,25, are given in the table below.

**Table**

| | | |
|---|---|---|
| | t_{ind} (s) | Cₑ (*10⁻⁴ M) |
| blank | 0 | 3.98 |
| 500 ppm carbamoylethyl-inulin; dp 10; ds 1.04 CONH₂ | 30 | 5.26 |
| 500 ppm carboxyethyl-carbamoylethyl-inulin; dp 30; ds 0.65 COO-; ds 0.10 CONH₂ | 1050 | 9.33 |
| 500 ppm carboxyethyl-carbamoylethyl-inulin; dp 10; ds 0.30 COO-; ds 0.24 CONH₂ | 200 | 7.24 |

### Example 8: Preparation of aminopropyl-nystose:

Nystose, a linear fructan consisting of 1 glucose unit and 3 fructose units, was cyanoethylated by etherification using acrylonitrile. Nystose (1 g, 6 mmol monomeric units) was dissolved in 1 ml water. Then a 5 % aqueous NaOH solution (960 mg, 1.2 mmol) and subsequently acrylonitrile (200 mg, 3.7 mmol) were added. The reaction mixture was heated at 45°C during 30 min. The reaction was stopped by neutralizing with 2 M HCl. The remaining acrylonitrile was evaporated under reduced pressure. Low molecular weight material (MW < 200) was removed by membrane filtration. Finally, the product was freeze-dried. Cyanoethyl-nystose with a ds of 0.56 was obtained (efficiency 91 %). In a second step the nitrile groups of cyanoethyl nystose were reduced into primary amino groups by hydrogenation with Raney cobalt as catalyst. Cyanoethyl nystose (0.5 g) was dissolved in 30 ml of a MeOH/H₂O mixture (90:10 v/v). The hydrogenation was carried out under H₂ pressure of 100 bar, a temperature 50°C, using 75 mg of the Raney cobalt catalyst. After 20 h, the catalyst was filtered off, the reaction mixture was neutralized and concentrated under reduced pressure. The product obtained by this means was a mixed cyanoethyl and aminopropyl ether of nystose (ds 0.26 and ds 0.21 for nitriles and amines, respectively).

### Example 9: Preparation of aminopropyl inulin using metals in liquid NH₃/MeOH:

Cyanoethyl-inulin with a ds=0.95 (1 g, 4.4 mmol cyanoethyl substituents) was dissolved in 75 ml of a mixture of dry MeOH and liq. NH₃ (1:2 (v/v)). Lithium (125 mg, 17.8 mmol) was added in small portions with stirring of the reaction mixture at - 45°C. After 90 min., the reaction mixture was brought to room temperature and NH₃ was allowed to evaporate. Some water was added carefully to the remaining solution, which was brought to neutral pH by bubbling through CO₂. In this way the dissolved NH₃ was converted to (NH₄)₂CO₃, which was removed by sublimation. Finally, low molecular weight material (MW < 200) was removed by membrane filtration and the product was freeze-dried. Aminopropyl-inulin with a ds of 0.70 (800 mg) was obtained.

### Example 10: Preparation of hydroxyamidinoethyl-inulin:

The hydroxyamidinoethyl derivative of inulin was prepared by reaction of the nitrile groups of cyanoethyl-inulin with NH₄OH. A solution of NH₄OH.HCl (1.8 g, 25 mmol) in 5 ml water was brought to pH=6.5 with a 2 N NaOH solution. Cyanoethyl-inulin with ds=0.95 (1 g, 4.4 mmol nitrile groups) was added and the reaction mixture was heated to 95°C for 5 h. The reaction product was purified using membrane filtration (yield: 950 mg) and was identified as hydroxyamidinoethyl inulin with ds=0.76. The structure of the reaction product was established using ¹³C NMR spectroscopy (δ (ppm) 32.0-33.0 (**C**H2C(=NOH)NH₂), 61.0-87.0 (C1, C3-C6 and O**C**H₂), 104.5-105.2 (C2), 157.0 - 158.0 (C(=NOH) NH₂). The ds was determined using ¹H NMR spectroscopy (δ (ppm) 2.75 - 2.85 (CH₂C(=NOH)NH₂), 3.3-4.5 (H1a-b, H3-H5, H6a-b and OCH₂), 5.3-5.5 (H1 of glucose unit).
The hydroxyamidinoethyl derivative of inulin is a complexant for transition metal ions. The Cu(II) complex has a green colour and shows an absorption maximum in the UV-Vis spectrum of λₘₐₓ=650 nm at pH 4.5 and λₘₐₓ=550 nm at pH 9.

## Claims

1. Fructan derivative **characterised in that**, for each monosaccharide unit, it contains 0.1 to 2.5 groups having the formula -CHR¹-CHR²-R³, wherein R¹ and R² each represent hydrogen or methyl and R³ represents -CN, -C(=NX)-NR⁴R⁵, -CO-NR⁴R⁵, -CO-OR⁴ or -CH₂-NR⁵R⁶, wherein R⁴ represents hydrogen or C₁-C₂₂-alkyl or C₁-C₂₂-alkenyl optionally substituted by hydroxy, R⁵ represents hydrogen, C₁-C₂₂-alkyl, carboxy- or hydroxy-substituted C₁-C₂₂ alkyl, or optionally substituted C₁-C₂₂-acyl, R⁶ represents hydrogen or C₁-C₂₂-acyl, and X represents hydrogen, hydroxyl or amino.

2. Fructan derivative according to claim 1, wherein R¹ and R² each represent hydrogen.

3. Fructan derivative according to claim 1 or 2, wherein R³ represents -C(=NOH)-NH₂, -CO-NR⁴H, -CO-OR⁴ or -CH₂₋NR⁵H, preferably -CONH₂ and/or -COOH.

4. Fructan derivative according to any one of claims 1-3, containing, for each monosaccharide unit, 0.2 to 1.5 groups having formula -CHR¹-CHR²-R³.

5. Fructan derivative according to any one of claims 1-4, wherein the fructan is inulin.

6. Process for producing a fructan derivative according to any one of claims 1-5, wherein fructan is reacted with a nitrile having formula R¹ HC=CR²-CN, with an amide having formula R¹ HC=CR²-CO-NR⁴R⁵, or with an ester having formula R¹HC=CR²-COOR⁷, wherein R¹, R², R⁴ and R⁵ are as defined in claim 1 and R⁷ represents hydrogen or C₁-C₄-alkyl, and the addition product is optionally hydrolysed, reduced, alkylated and/or oxidised.

7. Process according to claim 6, wherein the fructan is reacted with acrylonitrile and the addition product is reacted with hydrogen peroxide.

8. Process according to claim 6, wherein the fructan is reacted with acrylonitrile and the addition product is reacted with a borohydride or with hydrogen in the presence of a transition metal.

9. Process according to any one of claims 6-8, wherein the fructan is inulin.

10. Use of a fructan derivative according to any one of claims 1-5 for crystal growth inhibition.

11. Use of a fructan derivative according to any one of claims 1-5 as a chelating agent, an emulsifier, a dispersing agent, a surfactant, a hair fixative, a textile treatment aid or a contrast agent.

## Patentansprüche

1. Fructanderivat mit mindestens drei Monosaccharideinheiten, **dadurch gekennzeichnet, daß** es, pro jede Monosaccharideinheit, 0,1 bis 2,5 Gruppe(n) der Formel -CHR¹-CHR²-R³, worin R¹ und R² jeweils Wasserstoff oder Methyl darstellen und R³ für -CN, -C(=NX)-NR⁴R⁵, -CO-NR⁴R⁵, -CO-OR⁴ oder -CH₂-NR⁵R⁶ steht, worin R⁴ Wasserstoff oder C₁-C₂₂Alkyl oder C₁-C₂₂Alkenyl ggf. substituiert durch Hydroxy, darstellt, R⁵ für Wasserstoff, C₁-C₂₂Alkyl, carboxy- oder hydroxylsubstituiertes C₁-C₂₂Alkyl oder ggf. substituiertes C₁-C₂₂Acyl steht, R⁶ Wasserstoff oder C₁-C₂₂Acyl bedeutet und X Wasserstoff, Hydroxyl oder Amino entspricht, enthält.

2. Fructanderivat nach Anspruch 1, wobei R¹ und R² jeweils Wasserstoff darstellen.

3. Fructanderivat nach Anspruch 1 oder 2, wobei R³ -C(=NOH)-NH₂, -CO-NR⁴H, -CO-OR⁴ oder -CH₂-NR⁵H, vorzugsweise -CONH₂ und/oder -COOH bedeutet.

4. Fructanderivat nach einem der Ansprüche 1 bis 3, enthaltend, pro jede Monosaccharideinheit, 0,2 bis 1,5 Gruppe (n) der Formel -CHR¹-CHR²-R³.

5. Fructanderivat nach einem der Ansprüche 1 bis 4, wobei es sich bei dem Fructan um Inulin handelt.

6. Verfahren zur Herstellung eines Fructanderivats nach einem der Ansprüche 1 bis 5, wobei Fructan mit einem Nitril der Formel R¹HC=CR²-CN, mit einem Amid der Formel R¹HC=CR²-CO-NR⁴R⁵ oder mit einem Ester der Formel R¹HC=CR²-COOR⁷, worin R¹, R², R⁴ und R⁵ die in Anspruch 1 angegebene Bedeutung besitzen und R⁷ für Wasserstoff oder C₁-C₄Alkyl steht, umgesetzt und das Additionsprodukt ggf. hydrolysiert, reduziert, alkyliert und/oder oxidiert wird.

7. Verfahren nach Anspruch 6, wobei das Fructan mit Acrylnitril umgesetzt und das Additionsprodukt mit Wasserstoffperoxid reagieren gelassen werden.

8. Verfahren nach Anspruch 6, wobei das Fructan mit Acrylnitril umgesetzt und das Additionsprodukt mit Borhydrid oder mit Wasserstoff in Gegenwart eines Übergangsmetalls reagieren gelassen werden.

9. Verfahren nach einem der Ansprüche 6 bis 8, wobei es sich bei dem Fructan um Inulin handelt.

10. Verwendung eines Fructanderivats nach einem der Ansprüche 1 bis 5 zur Kristallwachstumshemmung.

11. Verwendung eines Fructanderivats nach einem der Ansprüche 1 bis 5 als Chelatbildner, Emulgator, Dispergiermittel, Netzmittel, Haarfixativ, Textilbehandlungshilfsmittel oder Kontrastmittel.

## Revendications

1. Dérivé de fructane ayant au moins trois motifs monosaccharide **caractérisé en ce que**, pour chaque motif monosaccharide, il contient 0,1 à 2,5 groupes de formule -CHR¹-CHR²-R³, dans laquelle R¹ et R² représentent chacun un atome d'hydrogène ou un groupe méthyle et R³ représente -CN, -C(=NX)-NR⁴R⁵, -CO-NR⁴R⁵, -CO-OR⁴ ou -CH₂-NR⁵R⁶, où R⁴ représente un atome d'hydrogène ou un groupe alkyle en C₁-C₂₂ ou alcényle en C₁-C₂₂ éventuellement substitué par un groupe hydroxy, R⁵ représente un atome d'hydrogène ou un groupe alkyle en C₁-C₂₂, alkyle en C₁-C₂₂ à substitution carboxy ou hydroxy ou acyle en C₁-C₂₂ éventuellement substitué, R⁶ représente un atome d'hydrogène ou un groupe acyle en C₁-C₂₂, et X représente un atome d'hydrogène ou un groupe hydroxyle ou amino.

2. Dérivé de fructane selon la revendication 1, dans lequel R¹ et R² représentent chacun un atome d'hydrogène.

3. Dérivé de fructane selon la revendication 1 ou 2, dans lequel R³ représente -C(=NOH)-NH₂, -CO-NR⁴H, -CO-OR⁴ ou -CH₂-NR⁵H, de préférence -CONH₂ et/ou -COOH.

4. Dérivé de fructane selon l'une quelconque des revendications 1-3, contenant, pour chaque motif monosaccharide, 0,2 à 1,5 groupe de formule -CHR¹-CHR²-R³.

5. Dérivé de fructane selon l'une quelconque des revendications 1-4, dans lequel le fructane est l'inuline.

6. Procédé de production d'un dérivé de fructane selon l'une quelconque des revendications 1-5, dans lequel le fructane est mis à réagir avec un nitrile de formule R¹HC=CR² -CN, avec un amide de formule R¹HC=CR²-CO-NR⁴R⁵, ou avec un ester de formule R¹HC=CR²-COOR⁷, où R¹, R², R⁴ et R⁵ sont tels que définis dans la revendication 1 et R⁷ représente un atome d'hydrogène ou un groupe alkyle en C₁-C₄, et le produit d'addition est éventuellement hydrolysé, réduit, alkylé et/ou oxydé.

7. Procédé selon la revendication 6, dans lequel le fructane est mis à réagir avec l'acrylonitrile et le produit d'addition est mis à réagir avec du peroxyde d'hydrogène.

8. Procédé selon la revendication 6, dans lequel le fructane est mis à réagir avec de l'acrylonitrile et le produit d'addition est mis à réagir avec un borohydrure ou avec de l'hydrogène en présence d'un métal de transition.

9. Procédé selon l'une quelconque des revendications 6-8, dans lequel le fructane est l'inuline.

10. Utilisation d'un dérivé de fructane selon l'une quelconque des revendications 1-5 pour inhiber la croissance cristalline.

11. Utilisation d'un dérivé de fructane selon l'une quelconque des revendications 1-5 en tant qu'agent chélatant, émulsifiant, agent dispersant, tensioactif, fixateur pour les cheveux, auxiliaire de traitement des textiles ou agent de contraste.
